Europäisches Patentamt

European Patent Office

Office européen des brevets

(11) Veröffentlichungsnummer: **0 417 804 A1**

# (12) EUROPÄISCHE PATENTANMELDUNG

(21) Anmeldenummer: 90117701.4

(22) Anmeldetag: 14.09.90

(51) Int. Cl.5: **C02F 1/46, C25B 1/00**

(30) Priorität: 15.09.89 DE 3930867

(43) Veröffentlichungstag der Anmeldung:
20.03.91 Patentblatt 91/12

(84) Benannte Vertragsstaaten:
AT BE CH DE DK ES FR GB GR IT LI LU NL SE

(71) Anmelder: **VEREINIGTE ALUMINIUM-WERKE AKTIENGESELLSCHAFT**
**Georg-von-Boeselager-Strasse 25**
**W-5300 Bonn 1(DE)**

(72) Erfinder: **Kudermann, Gerhard, Dr.**
**Henri-Spaak-Strasse 19**
**W-5305 Alfter(DE)**
Erfinder: **Blaufuss, Karl-Heinz**
**Günterscheider-Strasse 8**
**W-5469 Windhagen(DE)**

(74) Vertreter: **Müller-Wolff, Thomas, Dipl.-Ing.**
**c/o Vereinigte Aluminium-Werke AG**
**Patentabteilung Postfach 2468**
**W-5300 Bonn 1(DE)**

(54) **Verfahren zur anodischen Abscheidung von Mangan aus salzsauren Lösungen.**

(57) Das Verfahren ist dadurch gekennzeichnet, daß in der Lösung eine Konzentration an freier Säure von 0,001 bis 2 M eingestellt und das Mangan bei einer Spannung zwischen 1,3 V und 2,5 V anodisch als Mangan(IV)-Oxid abgeschieden wird.

Hiermit werden Mangangehalte in der Lösung von kleiner 10 mg/l ohne Verwendung einer organischen Extraktionslösung erreicht.

Anwendung des Verfahrens zur Herstellung von Polyaluminiumchloridlösungen für die Wasserreinigung, die Papierindustrie, als Hydrophobierungsmittel für Textilen, für Antispirantien und Deodorantien und als Keramikbinder.

EP 0 417 804 A1

## VERFAHREN ZUR ANODISCHEN ABSCHEIDUNG VON MANGAN AUS SALZSAUREN LÖSUNGEN.

Die Erfindung betrifft ein Verfahren zur Ausscheidung von Mangan aus salzsauren Lösungen. Unter salzsauren Lösungen verstehen wir auch Salzlösungen, bei denen durch Hydrolyse freie Salzsäure entsteht.

In DE 27 39 113 wird ein Verfahren beschrieben, mit dem Metallionen, unter anderem Mn, mit einem tertiären aliphatischen Amin in Kerosin extrahiert werden. Das Verfahren ist nur auf relativ hochkonzentrierte salzsaure Lösungen anwendbar, die einen Salzsäuregehalt von 12-18 Masse% aufweisen. Weniger konzentrierte Lösungen müssen mit konzentrierter Salzsäure erst auf diesen hohen Gehalt gebracht werden, bei dem die Extraktion gelingt (sie entspricht etwa 1:1 verdünnter Salzsäure).
Ferner werden bei diesem Verfahren benötigt:
Großes Volumen an Extraktionslösung, Reinigung und Rezyklierung des organischen Lösungsmittels (mehrstufiges Verfahren), Entsorgung der Waschwässer. Dabei können organische Spuren in die gereinigten sauren Lösungen gelangen (Probleme hinsichtlich Weiterverarbeitung zu hochwertigen Produkten) und weitere Verunreinigungen durch die hohen Salzsäuremengen eingetragen werden.

A.A. Yadav und S.M. Khopkar beschreiben in Separation Science, 4 (4), S. 349-355, 1969 ein Verfahren, mit dem sich Mn(II) mit Hilfe einer Extraktionslösung von 40 %-igem Tributylphosphat in Xylol extrahieren läßt aus mindestens 1 M salzsauren Lösungen, die 2,5 M Aluminiumchlorid oder 6 M Calciumchlorid als Aussalzreagenz enthalten.

Es zeigt sich damit, daß anstelle hoher Salzsäuremengen das Verteilungsgleichgewicht auch mit einem Aussalzreagenz in Richtung Extraktionslösung verschoben werden kann. Es ergeben sich jedoch ähnliche Nachteile wie oben bereits beschrieben, nämlich:
hoher Salzbedarf oder hoher Salzlösungskreislauf, Entsorgung der Waschwässer, Einschleppung von anorganischen und organischen Verunreinigungen in die sauren Lösungen.

Eine besondere Schwierigkeit ist die selektive Extraktion von Mn aus sauren Lösungen, die auch andere Metalle wie z.B. Al enthalten, die in der sauren Lösung verbleiben sollen. Es hat sich gezeigt, daß die Abscheidung von Metalloxiden durch Chlorid gestört wird, da die Abscheidepotentiale von Mn(IV)-Oxid und Chlorgas dicht beieinander liegen.

Aufgabe der vorliegenden Erfindung ist es, die beschriebenen Nachteile zu vermeiden und ein Verfahren zu entwickeln, mit dem nach der Behandlung Mangangehalte in der Lösung von kleiner 10 mg/l ohne Verwendung einer organischen Extraktionslösung erreicht werden. Die Aufgabe wird durch die in den Patentansprüchen angegebenen Merkmale gelöst.

Unsere Versuche belegen (Beispiel 1 - Trinkwasser), daß sich Mn in weitgehend salzfreien Lösungen, wie z.B. Trinkwasser bei pH 5,6 (Original Stadtwasser) und Spannungen von z.B. 7 und 14 V -wenn auch sehr langsam - anodisch abscheiden läßt. In Gegenwart von Chlorid oberhalb etwa 1 g/l wird anodisch vorwiegend $Cl_2$ und kein Mn abgeschieden.

Es wurde jedoch überraschenderweise festgestellt, daß bei Absenkung des pH-Wertes auf etwa 2 (Salzsäuregehalt 0,01 M) und Reduktion der Spannung auf etwa 2 V, auch über dem Oxidationspotential von Chlorid zu Chlorgas 1,36 V, die Abscheidung von Chlor minimal ist und Mangan als Mangan(IV)-Oxid anodisch abgeschieden wird.

Mit weiterer Erhöhung der Konzentration an freier Säure, nimmt die Abscheiderate von Mangan zu (Beispiele 2, 3 und 4). Die optimale Abscheiderate liegt zwischen einem pH-Wert von -0,2 (entsprechend 1,6 M Salzsäure) und pH 0,7 (entsprechend 0,2 M Salzsäure). Bei Chloridgehalten über 150 g/l gelingt die anodische Abscheidung von Mangan(IV)oxid auch unter diesen Bedingungen nicht mehr.

Unter "elektrochemischem Lösen" von Aluminium verstehen wir die Einbringung von metallischem Al als Komponente eines galvanischen Elements oder durch Anlegen einer äußeren Spannung zwischen zwei Elektroden, wobei eine Elektrode aus Aluminium besteht und als Anode geschaltet wird. Die Bildung eines galvanischen Elementes kann auch durch edlere Legierungskomponenten (z.B. Cu) im Aluminium oder durch Zugaben von Salzen edlerer Elemente als Al eingeleitet werden. Dadurch wird das Inlösungbringen des Metalls sehr beschleunigt. So beträgt beispielsweise die Zeit für das Inlösungbringen von 4,5 g Reinaluminium pro Liter Wasser ca 2 bis 3 h, während beim Anlegen von Strom dafür nur 30 min gebraucht werden und bei der Verwendung von Quecksilber oder Gallium als edlere Komponente in einem galvanischen Element sogar nur 15 min genügen. Andererseits ist es auch möglich, die Säurekonzentration durch die Verwendung von filterfeuchtem Aluminiumhydroxid einzustellen, das besonders schnell in Lösung geht und daher im Sinne einer optimalen Prozeßsteuerung vorteilhafter ist als normales handelsübliches Aluminiumhydroxid (Tab. 2). Wenn dabei die Menge des Hydroxids über 35 g/l liegt, ist es zweckmäßig, die Lösung vor der anodischen Abscheidung nochmals zu filtrieren, da auch geringe Mengen an ungelöstem Hydroxid den Abscheideprozeß stören können.

## Mn-Abscheidung aus chloridhaltigen Lösungen

Vergleichsbeispiel 1 (Trinkwasser)

Ein Becherglas wurde mit 500 ml Trinkwasser gefüllt und ein Mangangehalt von 5,0 mg/l eingestellt. Der pH-Wert betrug 5,6. Es wurde bei einer Spannung von 7 und 14 V anodisch Mangan(IV)-Oxid abgeschieden.

| Ergebnis: | | |
|---|---|---|
| Zeit min | Restgehalte Mn in mg/l | |
| | 7 V | 14 V |
| 0 | 5 | 5 |
| 30 | 3,36 | 4,45 |
| 60 | 2,26 | 2,18 |
| 120 | 0,91 | 0,90 |
| 180 | 0,79 | 0,77 |

Beispiel 2

In 250 ml salzsaurer Lösung (Al 30 g/l, $HCl_{frei}$ 65 g/l, $Cl_{ges.}$ 170 g/l, Mn 0,18 g/l) wurden 4,5 g Reinaluminium mit einem Kupfergehalt von etwa 0,004 Masse% gelöst und damit ein pH-Wert von etwa 0 erreicht. Die Lösung wurde mit Wasser auf 500 ml verdünnt. In die Lösung wurden 2 Platinelektroden getaucht, die mit einer regelbaren Gleichspannungsquelle verbunden wurden. Die wirksame Fläche der Platinanode betrug 75 $cm^2$. Die Lösung wurde mit einem Magnetrührer gerührt und eine Spannung von 2,5 V eingestellt. Bei dieser Spannung betrug die Chloridabscheidung 0,6 g/h. An der Anode begann sich Mangan-(IV)-Oxid abzuscheiden. Die Abscheidungsrate betrug 0,3 mg Mn/ min und nach etwa 2 Stunden wurde in der Lösung ein Mangangehalt von etwa 0,05 g/l erreicht. Der pH-Wert war auf etwa 0,7 angestiegen. Nach weiteren 2 Stunden betrug der Mangangehalt der Lösung noch etwa 0,015 g/l. Der pH-Wert wurde dabei konstant gehalten.

Beispiel 3

250 ml salzsaure Lösung mit einer Zusammensetzung wie in Beispiel 2 wurden mit 13 g $Al(OH)_3$ versetzt und bis zur vollständigen Lösung des $Al(OH)_3$, etwa 14 h, unter Rückfluß gekocht. Die Lösung wurde mit Wasser auf 500 ml verdünnt. Der pH-Wert betrug 0,2. Anschließend wurde wie in Beispiel 1, jedoch mit einer Spannung von 2,0 V Mangan(IV)-Oxid anodisch abgeschieden. Bei dieser Spannung betrug die Chloridabscheidung 0,4 g/h. Die Manganabscheidung betrug 0,5 mg Mn/min. Nach 2 Stunden wurde in der Lösung ein Mangangehalt von etwa 0,03 g/l erreicht. Nach weiteren 2 Stunden betrug der Mangangehalt noch 0,01 g/l. Der pH-Wert lag bei 0,7.

Beispiel 4

250 ml salzsaure Lösung mit einer Zusammensetzung wie in Beispiel 2 wurden in ein Becherglas gegeben, das 10 g geschmolzenes Gallium enthielt. In dia Lösung wurde ein Stab Reinaluminium so eingetaucht, daß er Kontakt zu dem geschmolzenen Gallium hatte. Das Reinaluminium begann sich zu lösen. Nach 2 Stunden wurde ein pH-Wert von 0 erreicht. Es haben sich etwa 4,5 g Al gelöst. Der Aluminiunstab wurde herausgenommen und die Lösung in ein weiteres Becherglas von Gallium abdekantiert und mit Wasser auf 500 ml verdünnt. Anschließend wurde wie in Beispiel 2 jedoch mit einer Spannung von 1,6 V Mangan(IV)-Oxid anodisch abgeschieden. Die Chlorentwicklung lag bei 0,2 g/h. Die Abscheidungsrate des Mangans betrug 0,55 mg Mn/min., und nach 2 Stunden wurde in der Lösung ein Mangangehalt von etwa 0,025 g/l erreicht. Nach weiteren 2 Stunden betrug der Mangangehalt weniger als 0,01 g/l. Der pH-Wert betrug 0,5.

Im folgenden wird das erfindungsgemäße Verfahren schematisch anhand eines Flußdiagramms erläutert. Es zeigt sich, daß bei einer Mn-Ausgangskonzentration von 0,17 g/l nach Durchführung der Verfahrensschritte a, b, c und der elektrolytischen Abscheidung von Mn(IV)-Oxid ein Mn-Gehalt in der Restlösung von unter 0,01 g/l verbleibt.

Elektrolytische Entfernung von Mangan aus salzsauren Lösungen

```
+----------------------------+
|Salzsaure Lösung 1000 l |
|Al              30    g/l|
|HCl(frei)       65    g/l|
|Cl(gesamt)     170    g/l|
|Mn             0,17 g/l|
+-----------+------------+
            |
+-----------+------------+
|Zulösen                 |
|a) Al(OH)3      52    g/l|
|b) Al elektr.   18    g/l|
|c) Al galvan.   18    g/l|
+-----------+------------+
            |
+-----------+------------+
|Verdünnen               |
|                        |
|+ 1000 l H2O            |
+-----------+------------+
            |
+-----------+------------+
|Elektrolytische         |
|Abscheidung von         |
|Mangan(IV)-oxid         |
+-----------+------------+
            |
+-----------+------------+
|Salzsaure Lösung 2000 l |
|Al              24    g/l|
|Cl(gesamt)      85    g/l|
|Mn             <0,01 g/l|
+----------------------------+
```

Tab. 1

| Zulösen von Aluminium bei einer Temperatur von 80 °C | | | | | |
|---|---|---|---|---|---|
| Zeit h | Methode | Al zugelöst g/l | pH | Al$_{ges}$ g/l | Cl$_{ges}$ g/l |
| 0,25 | Ga galv. | 4,5 | 0 | 19,5 | 80 |
| 0,25 | Hg galv. | 4,5 | 0 | 19,5 | 80 |
| 0,5 | Al mit 50 ppm Cu | 4,5 | 0 | 19,5 | 80 |
| 0,5 | anodisch | 4,5 | 0 | 19,5 | 807 |

Tab. 2

| Zulösen von Aluminiumhydroxid bei 120 °C | | | |
|---|---|---|---|
| Zeit h | Al(OH)$_3$ g/l | pH | Al$_{ges}$ g/l |
| 10 | 36 | -0,2 | 42,5 |
| 14 | 52 | 0,2 | 48 |

Die nach dem erfindungsgemäßen Verfahren hergestellten Polyaluminiumchloridlösungen können in der Wasserreinigung als Flockungsmittel, in der Papierindustrie, als Hydrophobierungsmittel für Textilien, als Keramikbinder sowie als Antispirantien und Deodorantien in der Körperpflege eingesetzt werden. Hierzu wird je nach dem Anwendungsgebiet nach dem Lösen in Wasser durch Zusatz von geeigneten Säuren und Salzen sowie auch durch Eindampfen der Lösungen das jeweils gewünschte Molverhältnis und die jeweils erforderliche Konzentration eingestellt.

Es ist besonders vorteilhaft, daß bereits beim Lösen des metallischen Aluminiums keine Salzsäure oder ACL$_3$-Lösung verwendet werden muß. Somit können Molverhältnisse von $m_{AL}/m_{Cl}$ bis zu 2 erreicht werden.

Für die genannten Anwendungsfälle ist es wichtig, daß eine sehr gute Homogenität der Produkte erreicht wird. Unter den erfindungsgemäßen Verfahrensbedingungen konnten keine wasserunlöslichen Anteile beobachtet werder.

**Ansprüche**

1. Verfahren zur Abscheidung von Mangan aus salzsauren Lösungen, dadurch gekennzeichnet, daß in der Lösung eine Konzentration an freier Säure von 0,01 bis 2 M eingestellt und das Mangan bei einer Spannung zwischen 1,3 V und 2,5 V anodisch als Mangan(IV)-Oxid abgeschieden wird.

2. Verfahren nach Anspruch 1, dadurch gekennzeichnet, daß zur Einstellung der freien Säurekonzentration von 0,01 bis 2 M metallisches Aluminium elektrochemisch zugelöst wird.

3. Verfahren nach einem der vorhergehenden Ansprüche, dadurch gekennzeichnet, daß zur Einstellung der freien Säurekonzentration von 0,01 M bis 2 M Aluminiumhydroxid bis zu Aluminiumgehalten von 48 g/l bis 70 g/l zugelöst wird.

4. Verfahren nach einem der vorhergehenden Ansprüche, dadurch gekennzeichnet, daß der Chloridgehalt der Lösung weniger als 150 g/l beträgt.

5. Verfahren nach einem der vorhergehenden Ansprüche, dadurch gekennzeichnet, daß die Lösung auf Temperaturen über 80 °C aufgeheizt und filterfeuchtes Aluminiumhydroxid zugelöst wird, nach Beendigung des Löseprozesses das ungelöste Aluminiumhydroxid abfiltriert und das Mn(IV)-Oxid anodisch abgeschieden wird.

6. Verfahren nach einem der vorhergehenden Ansprüche, dadurch gekennzeichnet, daß in der Lösung eine Konzentration an freier Säure von 0,2 M bis 1,6 M eingestellt wird.

7. Anwendung des Verfahrens nach einem der vorhergehenden Ansprüche 1 - 6 zur Herstellung von

EP 0 417 804 A1

Polyaluminiumchloridlösungen für die Wasserreinigung.

8. Anwendung des Verfahrens nach einem der Ansprüche 2 - 6 zur Herstellung von Polyaluminiumchloridlösungen für die Papierindustrie.

9. Anwendung des Verfahrens nach einem der vorhergehenden Ansprüche 3 - 6 zur Herstellung von Polyaluminiumchlorid als Hydrophobierungsmittel für Textilien.

10. Anwendung des Verfahrens nach einem der vorhergehenden Ansprüche 1 -6 zur Herstellung von Polyaluminiumchloridlösungen für Antispirantien und Deodorantien.

11. Anwendung des Verfahrens nach einem der vorhergehenden Ansprüche 2 - 6 zur Herstellung von Polyaluminiumchloridlösungen als Keramikbinder.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, Band 76, Nr. 6, 7. Februar 1972, Seite 349, linke Spalte, Zusammenfassung Nr. 30086u, Columbus, Ohio, US; L.N. DZHAPARIDZE et al.: "Production of active electrolytic manganese dioxide from manganese chloride", & PERERAB. MARGANTSEVYKH POLYMETAL. RUD GRUZ. 1970, S. 144-149; von REF. ZH. KHIM. 1970, Zusammenfassung Nr. 21L254<br>— — — | 1,4,6 | C 02 F 1/46<br>C 25 B 1/00 |
| X | CHEMICAL ABSTRACTS, Band 74, Nr. 20, 17. Mai 1971, Seite 468, rechte Spalte, Zusammenfassung Nr. 106618a, Columbus, Ohio, US;<br>& ZA-A-6 908 746 (MATSUSHITA ELECTRIC INDUSTRIAL) 24-08-1970<br>— — — | 1,4,6 | |
| A | DE-A-1 796 305  (MATSUSHITA ELECTRIC INDUSTRIAL)<br>* Ansprüche *<br>— — — | 1,4 | |
| A | US-A-4 707 227  (HIGGINS)<br>* Ansprüche *<br>— — — | 1,4 | |
| A | FR-A-1 486 434  (ETABLISSEMENTS DUFOUR ET IGON)<br>* Seite 1, linke Spalte, Zeile 32 - rechte Spalte, Zeile 2; Seite 2, linke Spalte, Zeilen 19-21; Anspr. *<br>— — — — — | 2 | RECHERCHIERTE SACHGEBIETE (Int. Cl.5)<br><br>C 02 F<br>C 25 B |

Der vorliegende Recherchenbericht wurde für alle Patentansprüche erstellt

| Recherchenort | Abschlußdatum der Recherche | Prüfer |
|---|---|---|
| Den Haag | 13 November 90 | KASPERS H.M.C. |